(19) Europäisches Patentamt
European Patent Office
Office européen des brevets

(11) Veröffentlichungsnummer : **0 052 842**
**B1**

(12) # EUROPÄISCHE PATENTSCHRIFT

(45) Veröffentlichungstag der Patentschrift : **18.09.85**

(21) Anmeldenummer : **81109667.6**

(22) Anmeldetag : **13.11.81**

(51) Int. Cl.⁴ : **C 07 C125/00**, C 07 C127/19, C 07 D209/86, C 07 D279/30// A01N47/28

(54) **N,N-Diarylcarbamoyl-fluoride, Verfahren zu ihrer Herstellung, sowie ihre Verwendung zur Herstellung von Harnstoffen und die dabei erhaltenen Harnstoffe.**

(30) Priorität : **25.11.80 DE 3044215**

(43) Veröffentlichungstag der Anmeldung : **02.06.82 Patentblatt 82/22**

(45) Bekanntmachung des Hinweises auf die Patenterteilung : **18.09.85 Patentblatt 85/38**

(84) Benannte Vertragsstaaten : **DE**

(56) Entgegenhaltungen :
**US-A- 3 671 218**
**CHEMICAL ABSTRACTS, Band 61, Nr. 1, 6. Juli 1964, Spalte 1, Zusammenfassung Nr. 3363a Columbus, Ohio, US H.P. METZGER et al.: "Evidence for an electrophilic mecanism in catalysis by hydrolytic enzymes"**
**CHEMICAL ABSTRACTS, Band 72, Nr. 15, 13. April 1970, Seite 35, Spalte 2, Zusammenfassung Nr. 74988m Columbus, Ohio, US J. BIETH et al.: "Photoregulation of biological activity by photochromic reagents. II. Inhibitors of acetylcholinesterase"**
**CHEMICAL ABSTRACTS, Band 77, Nr. 1, 3. Juli 1972, Seite 407, Spalte 2, Zusammenfassung Nr. 4501b Columbus, Ohio, US S.L. JOHNSON et al.: "Nucleophilic reactivity to diphenylcarbamoyl derivatives. Unimolecular nature of diphenylcarbamoyl chloride hydrolysis"**

(73) Patentinhaber : **BAYER AG**
**Konzernverwaltung RP Patentabteilung**
**D-5090 Leverkusen 1 Bayerwerk (DE)**

(72) Erfinder : **Baasner, Bernd, Dr.**
**Kalkstrasse 132**
**D-5090 Leverkusen 1 (DE)**
Erfinder : **Hagemann, Hermann, Dr.**
**Kandinskystrasse 52**
**D-5090 Leverkusen 1 (DE)**
Erfinder : **Klauke, Erich, Dr.**
**Eichendorffweg 8**
**D-5068 Odenthal (DE)**

**EP 0 052 842 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft N,N-Diarylcarbamoyl-fluoride, Verfahren zu ihrer Herstellung, sowie ihre Verwendung zur Herstellung von Harnstoffen und die dabei erhaltenen Harnstoffe.

N,N-Diphenylcarbamoyl-fluorid ist bekannt und kann nach Biochemistry *3*, 926 (1964) durch Umsetzung von N,N-Diphenylcarbamoyl-chlorid mit Antimontrifluorid in heißem Xylol, oder nach J. Org. Chem. *37*, 1383 (1972) durch Umsetzung von N,N-Diphenylcarbamoyl-pyridinium-chlorid mit Kaliumfluorid in 10 %igem Acetonitril hergestellt werden.

Diese Verfahren haben den Nachteil, daß sie aufwendig sind und N,N-Diphenylcarbamoyl-fluorid in mäßigen Ausbeuten liefern.

Es wurden neue N,N-Diarylcarbamoyl-fluoride der Formel

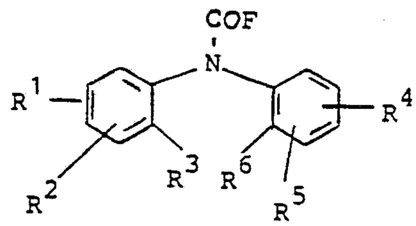

(I)

gefunden, in der

$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,

$R^2$, $R^3$, $R^5$ und $R^6$ unabhängig voneinander Wasserstoff, Halogen, Alkyl oder CN bedeuten und weiterhin

$R^3$ und $R^6$ gemeinsam auch eine einfache Bindung, Sauerstoff, Schwefel, $CH_2$, CO, oder Alkyliminoalkyliden bedeuten können,

wobei mindestens einer der Reste $R^1$ bis $R^6$ verschieden von Wasserstoff ist.

Bevorzugt sind N,N-Diarylcarbamoyl-fluoride der Formel

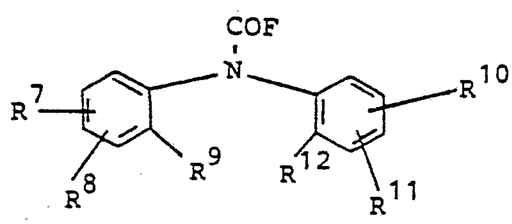

(II)

in der

$R^7$ und $R^{10}$ unabhängig voneinander Wasserstoff, $NO_2$, Halogen, Alkyl, Halogenalkyl, Aryl, mit Halogen und/oder $NO_2$ substituiertes Aryl, oder CN bedeuten,

$R^8$, $R^9$, $R^{11}$ und $R^{12}$ unabhängig voneinander Wasserstoff, Halogen oder Alkyl bedeuten und weiterhin

$R^9$ und $R^{12}$ gemeinsam auch eine einfache Bindung, Sauerstoff, Schwefel, $CH_2$ oder CO bedeuten, worin mindestens einer der Reste $R^7$ bis $R^{12}$ verschieden von Wasserstoff ist.

Als Halogen seien Fluor, Chlor und Brom, bevorzugt Fluor und Chlor, besonders bevorzugt Fluor genannt.

Als Alkyl seien beispielsweise geradkettige oder verzweigte Niederalkyle mit einem bis sechs C-Atomen wie Methyl, Ethyl, Propyl, Butyl, Pentyl, Isopropyl, Isobutyl, Hexyl und tert.-Butyl, bevorzugt Methyl und Ethyl genannt.

Als Halogenalkyl seien beispielsweise genannt Trifluormethyl und 2,2,2-Trifluorethyl, bevorzugt Trifluormethyl.

Als Aryl sei beispielsweise Phenyl und Naphthyl, bevorzugt Phenyl genannt.

Als Halogen für mit Halogen und/oder $NO_2$ substituiertes Aryl seien beispielsweise Fluor, Chlor und Brom, bevorzugt Fluor und Chlor genannt. Nitrogruppen sind ebenfalls bevorzugt.

Als mit Halogen und/oder $NO_2$ substituiertes Aryl seien beispielsweise 2-Chlorphenyl, 3-Chlorphenyl, 4-Chlorphenyl, 2,4-Dichlorphenyl, 2-Fluorphenyl, 4-Fluorphenyl, 2-Nitrophenyl, 4-Nitrophenyl, bevorzugt 2-Fluorphenyl, 4-Fluorphenyl und 4-Nitrophenyl genannt.

Als Dialkylamino seien beispielsweise Dimethylamino, Diethylamino, Dipropylamino, Methyl-ethylamino, Methyl-propylamino, Ethyl-propylamino, bevorzugt Dimethylamino, Diethylamino und Methylethylamino, besonders bevorzugt Diethylamino genannt.

Als Alkyliminoalkyliden seien beispielsweise Methylimino, Ethylimino, n-Propylimino, n-Butylimino, bevorzugt Ethylimino genannt.

Es wurde weiterhin ein Verfahren zur Herstellung von N,N-Diarylcarbamoyl-fluoriden der Formel

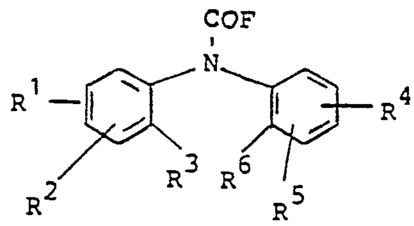

(Ia)

in der

$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,

$R^2$, $R^3$, $R^5$ und $R^6$ unabhängig voneinander Wasserstoff, Halogen, Alkyl oder CN bedeuten und weiterhin

$R^3$ und $R^6$ gemeinsam eine einfache Bindung, Sauerstoff, Schwefel, $CH_2$, CO oder Alkyliminoalkyliden bedeuten,

gefunden, das dadurch gekennzeichnet ist, daß man in einer ersten Stufe ein N,N-Diarylamin der Formel

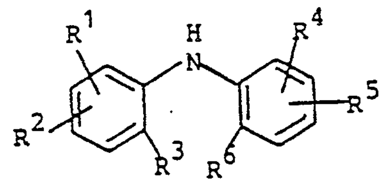

(III)

worin die Reste $R^1$ bis $R^6$ die bei Formel (Ia) angegebene Bedeutung haben,

mit Phosgen bei einer Temperatur von − 10 bis 250 °C, gegebenenfalls in einem inerten organischen Lösungsmittel, gegebenenfalls in Gegenwart von mindestens molaren Mengen einer basisch reagierenden Substanz, gegebenenfalls unter erhöhtem Druck, zum N,N-Diarylcarbamoyl-chlorid der Formel

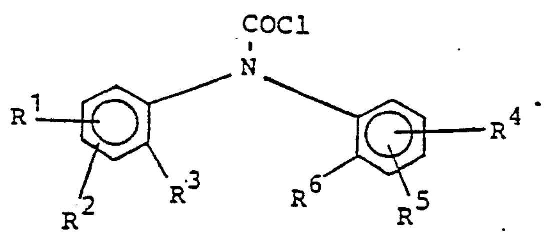

(IV)

worin die Reste $R^1$ bis $R^6$ die bei Formel (Ia) angegebene Bedeutung haben,

umsetzt und das Umsetzungsprodukt in einem zweiten Schritt mit einem Fluorierungsmittel in einem Lösungsmittel bei einer Temperatur von − 20 °C bis 300 °C, gegebenenfalls unter erhöhtem Druck, umsetzt, wobei für den Fall, daß ein $NO_2$-haltiges N,N-Diarylcarbamoylfluorid aus einem $NO_2$-freien oder -ärmeren Ausgangsmaterial hergestellt werden soll, nach der Fluorierung mit den üblichen Nitrierungsmitteln nitriert wird und wobei weiter für den Fall, daß ein chlor- oder bromhaltiges N,N-Carbamoylfluorid aus einem chlor- oder bromfreien oder -ärmeren Ausgangsmaterial hergestellt werden soll, nach der Fluorierung in einem inerten Lösungsmittel, gegebenenfalls in Gegenwart von Halogenüberträgern, chloriert oder bromiert wird.

Bei der Umsetzung des ersten Schrittes können beispielsweise 1 bis 10 Mol, bevorzugt 1 bis 2 Mol Phosgen pro Mol N,N-Diarylamin verwendet werden.

Für den Fall, daß in Gegenwart einer basisch reagierenden Substanz umgesetzt wird, können als solche beispielsweise gegenüber N,N-Diarylcarbamoylchloriden inerte tertiäre Amine wie Pyridin eingesetzt werden.

Für den Fall, daß in Gegenwart eines Lösungsmittels gearbeitet werden soll, können beispielsweise aliphatische Kohlenwasserstoffe wie Pentan, Hexan, Heptan, Cyclohexan, halogenierte aliphatische Kohlenwasserstoffe wie Dichlormethan, Trichlormethan, Tetrachlormethan, aromatische und substituierte aromatische Kohlenwasserstoffe wie Benzol, Toluol, Xylol, Chlorbenzolund Dichlorbenzole eingesetzt werden. Bevorzugt werden aromatische und substituierte aromatische Kohlenwasserstoffe wie Benzol, Toluol, Xylol, Chlorbenzol und Dichlorbenzole, besonders bevorzugt Toluol, Xylol und Chlorbenzol eingesetzt.

Die Umsetzung kann vorzugsweise bei Temperaturen von 0 bis 200 °C, besonders bevorzugt von 20 bis 150 °C, durchgeführt werden.

Für den Fall, daß die Umsetztung bei erhöhtem Druck ausgeführt werden soll, kann bei einem Druck von 1 bis 20 bar, bevorzugt bei 1 bis 2 bar, gearbeitet werden. Es ist jedoch bevorzugt, bei Normaldruck zu arbeiten.

Das erfindungsgemäße Verfahren kann beispielsweise so durchgeführt werden, daß in eine Lösung des N,N-Diarylamins in einem inerten organischen Lösungsmittel im angegebenen Temperatur- und Druckbereich Phosgen eingeleitet wird. Es kann aber auch so durchgeführt werden, daß eine Lösung von N,N-Diarylamin in einem inerten organischen Lösungsmittel zu einer Lösung von Phosgen in einem inerten organischen Lösungsmittel gegeben wird.

Bei der Umsetzung des zweiten Schrittes können als Fluorierungsmittel beispielsweise wasserfreier Fluorwasserstoff, Alkalifluoride wie Lithiumfluorid, Natriumfluorid, Natriumhydrogenfluorid, Kaliumfluorid, Kaliumhydrogenfluorid, Erdalkalifluoride wie Magnesium- und Calciumfluorid, Fuoride weiterer Metalle, wie Antimontri- Antimonpentafluorid verwendet werden. Bevorzugt seien wasserfreier Fluorwasserstoff, Natriumfluorid, Natriumhydrogenfluorid, Kaliumfluorid, Kaliumhydrogenfluorid, Magnesiumfluorid, Antimontrifluorid, besonders bevorzugt wasserfreier Fluorwasserstoff genannt.

Selbstverständlich können auch Mischungen der genannten Fluorierungsmittel verwendet werden.

Werden Metallfluoride als Fluorierungsmittel eingesetzt, können hiervon beispielsweise 1 bis 100 Mol, bevorzugt 1 bis 10 Mol, besonders bevorzugt 1 bis 3 Mol pro Mol N,N-Diarylcarbamoylchlorid eingesetzt werden, wobei die Fluorierung beispielsweise im Temperaturbereich von 20 bis 300 °C, bevorzugt 40 bis 250 °C, besonders bevorzugt von 80 bis 250 °C, durchgeführt werden kann und die Umsetzung bei einem Druck von 1 bis 30 bar, bevorzugt bei Normaldruck durchgeführt werden kann.

Wird wasserfreier Fluorwasserstoff als Fluorierungsmittel eingesetzt, kann hiervon beispielsweise 1 bis 200 Mol, bevorzugt 1 bis 100 Mol, besonders bevorzugt 1 bis 50 Mol pro Mol N,N-Diarylcarbamoylchlorid eingesetzt werden, wobei die Fluorierung im Temperaturbereich von – 20 bis 300 °C, bevorzugt 0 bis 250 °C, besonders bevorzugt 20 bis 100 °C durchgeführt werden kann und die Umsetzung bei einem Druck zwischen 1 bis 50 bar, bevorzugt 1 bis 30 bar ausgeführt werden kann.

Als Lösungsmittel für die Fluorierung können beispielsweise wasserfreier Fluorwasserstoff oder inerte organische Lösungsmittel verwendet werden. Beispielsweise seien genannt: Ether, wie Tetrahydrofuran und Dioxan, Polyethylenglykolether wie Ethylenglykoldimethylether, Ethylenglykoldiethylether, Diether des Tri- und Tetraethylenglykols, Sulfoxide, wie Dimethylsulfoxid, Diethylsulfoxid, Dimethylsulfon, Diethylsulfon, Tetramethylensulfon, N,N-disubstituierte Amide, wie Dimethylformamid, N,N-Dimethylacetamid, N,N-Diethylacetamid, N-Methylpyrrolidon, Hexamethylphosphorsäuretriamid, aromatische Kohlenwasserstoffe, wie Benzol, halogenierte aromatische Kohlenwasserstoffe, wie Chlorbenzol und Dichlorbenzole.

Bevorzugt wird Fluorwasserstoff, Ethylenglykoldimethylether, Ethylenglykoldiethylether, Tetramethylensulfon, Dimethylformamid, N,N-Diethylacetamid, Chlorbenzol und Dichlorbenzole, bei Verwendung von Fluorwasserstoff auch Dichlormethan, besonders bevorzugt Fluorwasserstoff, Tetramethylensulfon, Dimethylformamid, Chlorbenzol und Dichlorbenzole eingesetzt.

Selbstverständlich können auch Mischungen der genannten Lösungsmittel eingesetzt werden.

Die bei diesen Verfahren eingesetzten N,N-Diaryl-amine können beispielsweise nach C. Ferri, Reaktionen in der organischen Synthese, Georg-Thieme-Verlag, Stuttgart, 1978, S. 496 hergestellt werden, indem man N,N-Diarylcarbonsäureamide zu den entsprechenden N,N-Diarylaminen verseift.

Dieses oben beschriebene erfindungsgemäße Verfahren kann beispielsweise so durchgeführt werden, daß das N,N-Diarylcarbamoyl-chlorid, gelöst in einem inerten organischen Lösungsmittel, zum vorgelegten Fluorierungsmittel gegeben wird, es kann aber auch zweckmäßig sein, umgekehrt zu verfahren.

Dieses erfindungsgemäße Verfahren kann beispielsweise so ausgeführt werden :

In einem ersten Schritt wird das N,N-Diarylamin und Phosgen in einer der oben beschriebenen Reihenfolge, gegebenenfalls in Gegenwart einer basisch reagierenden Substanz, gegebenenfalls in einem Lösungsmittel, im angegebenen Temperatur- und Druckbereich zusammengegeben. Nach Abtrennung von eventuell ausfallendem Aminhydrochlorid und Beseitigung von eventuell überschüssigem Phosgen können die N,N-Diarylcarbamoyl-chloride nach einer der üblichen Methoden, beispielsweise Kristallisation, Destillation und/oder Chromatographie isoliert und/oder gereinigt werden.

In einem zweiten Schritt werden das N,N-Diarylcarbamoyl-chlorid, gelöst in einem inerten organischen Lösungsmittel, und das Fluorierungsmittel in einer der oben beschriebenen Reihenfolgen zusammengegeben und auf die Umsetzungstemperatur gebracht. Für den Fall, daß wasserfreier Fluorwasserstoff als Fluorierungsmittel verwendet wird, und die Umsetzungstemperatur oberhalb des Siedepunktes von wasserfreiem Fluorwasserstoff liegt, wird in einem Druckgefäß gearbeitet. Überschüssiges Fluorierungsmittel wird nach der Umsetzung, beispielsweise durch Destillation und/oder Filtration abgetrennt. Die Isolierung und Reinigung der N,N-Diarylcarbamoyl-fluoride kann nach einer der üblichen Methoden, beispielsweise Destillation, Kristallisation und/oder einem chromatographischen Verfahren erfolgen.

Es wurde ein weiteres Verfahren zur Herstellung von N,N-Diaryl-carbamoyl-fluoriden der Formel

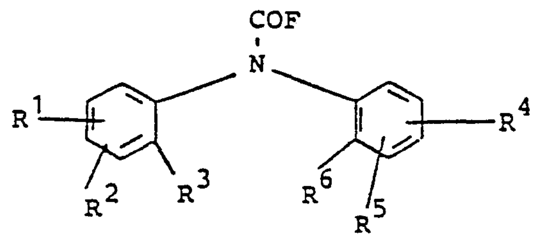

(Ia)

in der

$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,

$R^2$, $R^3$, $R^5$ und $R^6$ unabhängig voneinander Wasserstoff, Halogen, Alkyl oder CN bedeuten und weiterhin

$R^3$ und $R^6$ gemeinsam eine einfache Bindung, Sauerstoff, Schwefel, $CH_2$, CO oder Alkyliminoalkyliden bedeuten,

gefunden, das dadurch gekennzeichnet ist, daß man N,N-Diarylamine der Formel

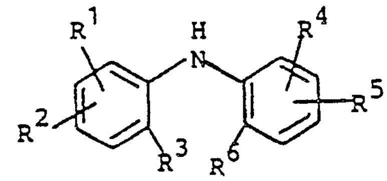

(III)

worin die Reste $R^1$ bis $R^6$ die bei Formel (Ia) angegebene Bedeutung haben, mit einem Fluorphosgen bei einer Temperatur von − 10 bis 250 °C, gegebenenfalls in einem inerten organischen Lösungsmittel, gegebenenfalls in Gegenwart von mindestens molaren Mengen einer basisch reagierenden Substanz, gegebenenfalls unter erhöhtem Druck umsetzt, wobei für den Fall, daß ein $NO_2$-haltiges N,N-Diarylcarbamoylfluorid aus einem $NO_2$-freien oder -ärmeren Ausgangsmaterial hergestellt werden soll, nach der Umsetzung mit den üblichen Nitrierungsmitteln nitriert wird und wobei weiter für den Fall, daß ein chlor- oder bromhaltiges N,N-Carbamoyl-fluorid aus einem chlor- oder bromfreien oder -ärmeren Ausgangsmaterial hergestellt werden soll, nach der Umsetzung in einem inerten Lösungsmittel, gegebenenfalls in Gegenwart von Halogenüberträgern, chloriert oder bromiert wird.

Fluorphosgen kann erfindungsgemäß Monofluoro- und Difluorophosgen sein

Bei der Umsetzung können beispielsweise 1 bis 10 Mol, bevorzugt 1 bis 2 Mol Fluorphosgen pro Mol N,N-Diarylamin verwendet werden.

Fluorphosgen kann beispielsweise nach J. Am. Chem. Soc. *79*, 5802 (1957) durch Fluorierung von Phosgen mit Fluorwasserstoff hergestellt werden.

Als basisch reagierende Substanzen seien beispielsweise inerte tertiäre Amine wie Pyridin genannt.

Als Lösungsmittel können beispielsweise aliphatische Kohlenwasserstoffe wie Pentan, Hexan, Heptan, Cyclohexan, halogenierte aliphatische Kohlenwasserstoffe wie Dichlormethan, Trichlormethan, Tetrachlormethan, aromatische und substituierte aromatische Kohlenwasserstoffe wie Benzol, Toluol, Xylol, Chlorbenzol, Dichlorbenzole eingesetzt werden. Bevorzugt werden aromatische und substituierte aromatische Kohlenwasserstoffe wie Benzol, Toluol, Xylol, Chlorbenzol, Dichlorbenzole besonders bevorzugt Toluol, Xylol und Chlorbenzol eingesetzt.

Die Umsetzung kann beispielsweise bei Temperaturen von 0 bis 200 °C, bevorzugt von 20 bis 150 °C durchgeführt werden.

Die Umsetzung kann beispielsweise bei einem Druck von 1 bis 20 bar, bevorzugt bei 1 bis 2 bar, besonders bevorzugt bei Normaldruck durchgeführt werden.

Das erfindungsgemäße Umsetzten mit Fluorphosgen kann beispielsweise so durchgeführt werden, daß in eine Lösung des N,N-Diarylamins in einem inerten organischen Lösungsmittel im angegebenen Temperatur- und Druckbereich Fluorphosgen eingeleitet wird. Es kann aber auch so durchgeführt werden, daß eine Lösung von N,N-Diarylamin in einem inerten organischen Lösungsmittel zu einer Lösung von Fluorphosgen in einem inerten Lösungsmittel gegeben wird.

Bei dieser erfindungsgemäßen Umsetzung kann beispielsweise so verfahren werden :

N,N-Diarylamin und Fluorphosgen werden in einer der oben beschriebenen Reihenfolgen, gegebenenfalls in Gegenwart einer basisch reagierenden Substanz, gegebenenfalls in einem Lösungsmittel, im angegebenen Temperatur- und Druckbereich zusammengegeben. Nach Abtrennung von eventuell ausfallendem Aminhydrochlorid und Beseitigung von eventuell überschüssigem Fluorphosgen können die N,N-Diarylcarbamoyl-fluoride nach einer der üblichen Methoden, beispielsweise Kristallisation, Destillation und/oder Chromatographie isoliert und/oder gereinigt werden.

Es wurde ein weiteres Verfahren zur Herstellung von N,N-Diarylcarbamoyl-fluoriden der Formel (Ia) gefunden, das dadurch gekennzeichnet ist, daß man in einem ersten Schritt N-Trichlormethyl-N,N-diarylamine der Formel

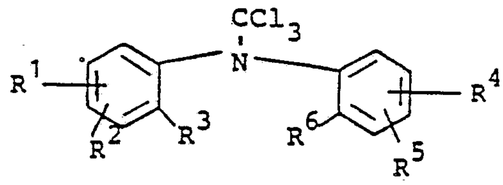

(V)

in der die Reste $R^1$ bis $R^6$ die bei Formel (Ia) angegebene Bedeutung besitzen, gegebenenfalls in Gegenwart eines inerten organischen Lösungsmittels, in einem Temperaturbereich von − 20 °C bis 100 °C mit Wasser umsetzt und in einer zweiten Stufe mit einem Fluorierungsmittel in einem Lösungsmittel bei einer Temperatur von − 20 °C bis 300 °C, gegebenenfalls unter erhöhtem Druck, umsetzt, wobei für den Fall, daß ein $NO_2$-haltiges N,N-Diarylcarbamoylfluorid aus einem $NO_2$-freien oder -ärmeren Ausgangsmaterial hergestellt werden soll, nach der Fluorierung mit den üblichen Nitrierungsmitteln nitriert wird und wobei weiter für den Fall, daß ein chlor- der bromhaltiges N,N-Carbamoylfluorid aus einem chlor- oder bromfreien oder -ärmeren Ausgangsmaterial hergestellt werden soll, nach der Fluorierung in einem inerten Lösungsmittel, gegebenenfalls in Gegenwart von Halogenüberträgern, chloriert oder bromiert wird.

Pro Mol N-Trichlormethyl-N,N-diarylamin kann beispielsweise 1 bis 10 Mol Wasser, bevorzugt etwa ein Mol Wasser eingesetzt werden.

Die Umsetzung mit Wasser wird bevorzugt bei − 10 bis 50 °C durchgeführt.

Als inerte organische Lösungsmittel seien beispielsweise Dichlormethan, Tetrachlormethan, Ether wie Diethylether, Tetrahydrofuran und Dioxan, aromatische Kohlenwasserstoffe wie Benzol, Toluol, Chlorbenzol und Dichlorbenzole, aliphatische Kohlenwasserstoffe wie Cyclohexan, sowie Essigster und Aceton, bevorzugt Dichlormethan, Tetrahydrofuran, Dioxan und Aceton genannt.

Dieses erfindungsgemäße zweistufige Vergafren kann beispielsweise so durchgeführt werden, daß man die N-Trichlormethyl-N,N-diarylamine in einem organischen Lösungsmittel vorlegt und dazu im angegebenen Temperaturbereich das Wasser, gegebenenfalls mit einem organischen Lösungsmittel verdünnt, gibt. Es kann aber auch umgekehrt verfahren werden.

Die als Zwischenprodukte entstehenden N,N-Diarylcarbamoyl-chloride können nach einem üblichen Verfahren, beispielsweise durch Kristallisation isoliert und/oder gereinigt werden und sodann der Fluorierung, beispielsweise unter den obengenannten Bedingungen, unterzogen werden.

Die im erfindungsgemäßen Verfahren eingesetzten N-Tri-chlormethyl-N,N-diarylamine können nach einem bisher nicht beschriebenen Verfahren hergestellt werden, das dadurch gekennzeichnet ist, daß man

a) N,N-Diarylamine mit Ameisensäure in einem Temperaturbereich bei 30 °C bis 150 °C, gegebenenfalls unter erhöhtem Druck, gegebenenfalls in Gegenwart eines inerten organischen Lösungsmittels, gegebenenfalls in Gegenwart eines wasserbindenden Mittels, umsetzt,

b) die erhaltenen N,N-Diarylformamide der Formel

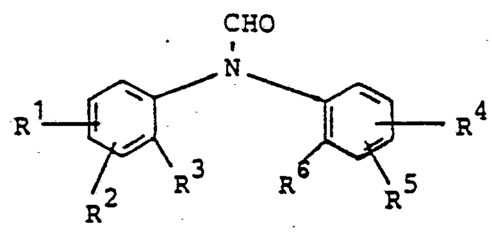

(VII)

worin die Reste $R^1$ bis $R^6$ die bei Formel (Ia) angegebene Bedeutung haben, mit einer schwefelliefernden Substanz in Gegenwart einer basisch reagierenden Substanz in einem inerten Lösungsmittel bei einer Temperatur von 50 °C bis 180 °C, umsetzt.

c) die dabei erhaltenen N,N-Diaryl-thioformamide der Formel

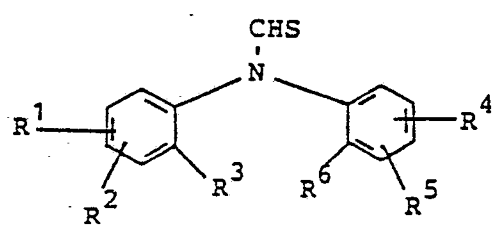

(VI)

in der $R^1$ bis $R^6$ die bei Formel (Ia) angegebene Bedeutung haben, mit elementarem Chlor in einem inerten organischen Lösungsmittel, in einem Temperaturbereich von − 20 °C bis 250 °C chloriert.

Bevorzugt wird die Chlorierung bei 40 bis 180 °C, besonders bevorzugt bei 60 bis 140 °C durchgeführt.

Die Stufe b) dieses Verfahrens kann analog an sich bekannter Methoden, beispielsweise nach Houben-Weyl, Bd. IX, S. 764 (1955) erfolgen, indem die N,N-Diarylformamide mit schwefelliefernden Substanzen, wie den Sulfiden des Phosphors, Aluminiumsulfid, Kaliumsulfid, Kaliumhydrogensulfid, Natriumsulfid, Natriumhydrogensulfid, Ammoniumsulfid oder Gemischen dieser Sulfide, am vorteilhaftesten den Sulfiden des Phosphors, in inerten organischen Lösungsmitteln umgesetzt werden.

Als Lösungsmittel für die Stufen a), b) und c) können beispielsweise Toluol, Xylole, und Pyridin, bevorzugt Pyridin, eingesetzt werden.

Die Umsetzung kann im Temperaturbereich von 50 bis 180 °C, vorzugsweise 80 bis 130 °C bei Normaldruck durchgeführt werden.

Es wurde ein weiteres Verfahren zur Herstellung von N,N-Diarylcarbamoyl-fluoriden der Formel (Ia) gefunden, das dadurch gekennzeichnet ist, daß man N-Trichlormethyl-N,N-di-arylformamide mit einem Fluorierungsmittel, gegebenenfalls in einem inerten organischen Lösungsmittel, gegebenenfalls bei erhöhter Temperatur, gegebenenfalls unter erhöhtem Druck, zu N-Dichlorfluormethyl-N,N-di-arylaminen der Formel

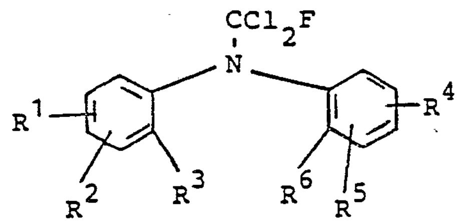

(VIII)

in der

$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,

$R^2$, $R^3$, $R^5$ und $R^6$ unabhängig voneinander Wasserstoff, Halogen, Alkyl oder CN bedeuten und weiterhin

$R^3$ und $R^6$ gemeinsam auch eine einfache Bindung, Sauerstoff, Schwefel, $CH_2$, CO, oder Alkyliminoalkyliden bedeuten können, fluoriert und in einem zweiten Reaktionsschritt, gegebenenfalls in einem Lösungsmittel, bei einer Temperatur von − 20 bis 100 °C mit Wasser umsetzt, wobei für den Fall, daß ein $NO_2$-haltiges N,N-Diarylcarbamoyl-fluorid aus einem $NO_2$-freien oder -ärmeren Ausgansmaterial hergestellt werden soll, nach der Umsetzung mit einem üblichen Nitrierungsmittel nitriert wird und wobei für den Fall, daß ein chlor- oder bromhaltiges N,N-Carbamoyl-fluorid aus einem chlor- oder bromfreien oder -ärmeren Ausgangsmaterial hergestellt werden soll, nach der Umsetzung in einem inerten Lösungsmittel, gegebenenfalls in Gegenwart von Halogenüberträgern, chloriert oder bromiert wird.

Als Fluorierungsmittel für dieses Verfahren können beispielsweise wasserfreier Fluorwasserstoff, Alkalifluoride wie Lithiumfluorid, Natriumfluorid, Natriumhydrogenfluorid, Kaliumfluorid und Kaliumhydrogenfluorid, Erdalkalifluoride wie Magnesium- und Calciumfluorid, Fluoride anderer Metalle, wie Antimontri- und Antimonpentafluorid eingesetzt werden. Bevorzugt seien wasserfreier Fluorwasserstoff, Natriumfluorid, Natriumhydrogenfluorid, Kaliumfluorid, Kaliumhydrogenfluorid, Magnesiumfluorid, Antimontrifluorid, besonders bevorzugt wasserfreier Fluorwasserstoff genannt.

Selbstverständlich können auch Mischungen der Fluorierungsmittel eingesetzt werden.

Werden Metallfluoride bei dieser Fluorierung als Fluorierungsmittel eingesetzt, können 1 bis 100 Mol, bevorzugt 1 bis 10 Mol, besonders bevorzugt 1 bis 3 Mol pro Mol N-Trichlormethyl-N,N-diarylamin eingesetzt werden, wobei die Fluorierung im Temperaturbereich von beispielsweise 20 bis 300 °C, bevorzugt 40 bis 250 °C, besonders bevorzugt von 80 bis 250 °C, durchgeführt werden kann und die Umsetzung bei einem Druck von beispielsweise 1 bis 30 bar, bevorzugt bei Normaldruck durchgeführt werden kann.

Wird wasserfreier Fluorwasserstoff bei dieser Fluorierung als Fluorierungsmittel eingesetzt, kann beispielsweise 1 bis 1,5 Mol, bevorzugt 1 bis 1,2 Mol, besonders bevorzugt 1 bis 1,05 Mol pro Mol N-Trichlormethyl-N,N-diarylamine eingesetzt werden, wobei die Fluorierung im Temperaturbereich von beispielsweise − 20 bis 300 °C, bevorzugt 0 bis 250 °C, besonders bevorzugt 20 bis 100 °C durchgeführt werden kann und die Umsetzung bei einem Druck von beispielsweise 1 bis 50 bar, bevorzugt 1 bis 30 bar ausgeführt werden kann.

Als Lösungsmittel bei der Fluorierung mit einem Fluorierungsmittel können beispielsweise wasserfreier Fluorwasserstoff oder inerte organische Lösungsmittel eingesetzt werden. Beispielsweise seien genannt : Ether, wie Tetrahydrofuran und Dioxan, Polyethylenglykolether wie Ethylenglykoldimethylether und Ethylenglykoldiethylether, Diether des Tri- und Tetraethylenglykols, Sulfoxide, wie Dimethylsulfoxid, Diethylsulfoxid, Dimethylsulfon, Diethylsulfon und Tetramethylensulfon, N,N-disubstituierte Amide, wie Dimethylformamid, N,N-Dimethylacetamid, N,N-Diethylacetamid, N-Methylpyrrolidon und Hexamethylphosphorsäuretriamid, aromatische Kohlenwasserstoffe, wie Benzol, halogenierte aromatische Kohlenwasserstoffe, wie Chlorbenzol und Dichlorbenzole.

Bevorzugt wird Fluorwasserstoff, Ethylenglykoldimethylether, Ethylenglykoldiethylether, Tetramethylensulfon, Dimethylformamid, N,N-Diethylacetamid, Chlorbenzol und Dichlorbenzole, bei

Verwendung von Fluorwasserstoff auch Dichlormethan. Besonders bevorzugt werden Fluorwasserstoff, Tetramethylensulfon, Dimethylformamid, Chlorbenzol und Dichlorbenzole eingesetzt.

Die Umsetzung mit Wasser kann beispielsweise bei − 20 bis 100 °C, bevorzugt bei − 10 bis 50 °C durchgeführt werden.

Es können beispielsweise 1 bis 10 Mol, bevorzugt etwa 1 Mol Wasser eingesetzt werden.

Als inerte organische Lösungsmittel für die Umsetzung mit Wasser seien beispielsweise Dichlormethan, Tetrachlormethan, Ether wie Diethylether, Tetrahydrofuran und Dioxan, aromatische Kohlenwasserstoffe wie Benzol, Toluol, Chlorbenzol, Dichlorbenzole, aliphatische Kohlenwasserstoffe wie Cyclohexan sowie Essigester und Aceton genannt. Bevorzugt seien Dichlormethan, Tetrahydrofuran, Dioxan und Aceton genannt.

Die erfindungsgemäße Fluorierung und Umsetzung mit Wasser kann beispielsweise so durchgeführt werden, daß in einem ersten Schritt N-Trichlormethyl-N,N-diarylformamide, gegebenenfalls in einem Lösungsmittel in einer der oben angegebenen Reihenfolgen, mit dem Fluorierungsmittel zusammengebracht und auf die gewünschte Umsetzungstemperatur gebracht werden. Nach Entfernung des gegebenenfalls anwesenden Lösungsmittels können die gewonnenen N-Dichlorfluormethyl-N,N-diarylamine, beispielsweise durch Destillation, Kristallisation und/oder Chromatographie, gereinigt werden. Es kann jedoch auch zweckmäßig sein, sie nicht weiter zu reinigen.

Im zweiten Schritt des erfindungsgemäßen Verfahrens wird das N-Dichlorfluormethyl-N,N-diarylamin, gegebenenfalls in einem Lösungsmittel, im oben angegebenen Temperaturbereich mit Wasser umgesetzt. Nach Entfernung des gegebenenfalls anwesenden Lösungsmittels können die erhaltenen N,N-Diarylcarbamoylfluoride nach bekannten Methoden, beispielsweise Kristallisation, Destillation und/oder Chromatographie, gereinigt werden.

Für den Fall, daß beispielsweise ein N,N-Diarylcarbamoylfluorid hergestellt werden soll, das mehr Nitrogruppen enthalten soll als das Ausgangsmaterial, können die Nitrogruppen durch Nitrierung des N,N-Diarylcarbamoylfluorids eingeführt werden.

So können beispielsweise N,N-Diarylcarbamoylfluoride der Formel (Ia) gegebenenfalls in einem Lösungsmittel mit einem üblichen Nitrierungsmittel zu Nitrogruppen haltigeren N,N-Diarylcarbamoylfluoriden nitriert werden.

Als Nitrierungsmittel können beispielsweise mit Eisessig verdünnte Salpetersäure, Gemische aus Metallnitraten und Säure, beispielsweise Kaliumnitrat und Schwefelsäure, oder Nitriersäuregemische, beispielsweise Schwefelsäure und Salpetersäure eingesetzt werden.

Für den Fall, daß beispielsweise ein N,N-Diarylcarbamoylfluorid hergestellt werden soll, das mehr Halogene enthalten soll als das Ausgangsmaterial, können die Halogene durch Halogenierung des N,N-Diarylcarbamoylfluorids, gegebenenfalls in einem Lösungsmittel mit einem Halogenierungsmittel eingeführt werden.

Als Halogenierungsmittel seien beispielsweise Halogene wie Chlor und Brom und Halogenüberträger wie Sulfurylchlorid, Sulfurylbromid und Phosphorpentachlorid genannt.

Die Erfindung betrifft weiterhin die Verwendung von N,N-Diaryl-carbamoylfluoriden der Formel (I), die eine oder mehr Nitrogruppen enthalten, zur Herstellung von Harnstoffen der Formel

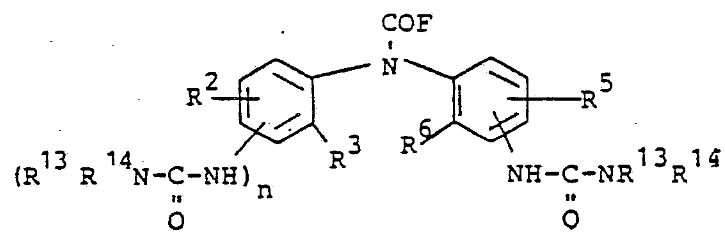

(IX)

in der die Reste $R^2$, $R^3$, $R^5$ und $R^6$ die bei Formel (I) angegebene Bedeutung haben,

$R^{13}$ und $R^{14}$ unabhängig voneinander für Wasserstoff, Alkyl, Halogenalkyl, Aryl, mit Halogen und/oder $NO_2$ substituiertes Aryl stehen und

n für 0 oder 1 steht, in dem man in einer ersten Stufe nitrogruppenhaltige N,N-Diarylcarbamoylfluoride der Formel (I) gegebenenfalls in Gegenwart eines organischen Lösungsmittels, in Gegenwart eines geeigneten Hydrierungskatalysators mit Wasserstoff zu aminogruppenhaltigen N,N-Diarylcarbamoylfluoriden der Formel

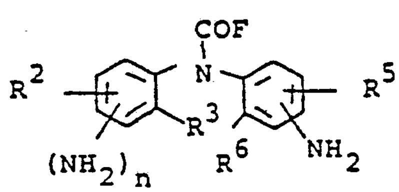

(X)

in der die Reste $R^2$, $R^3$, $R^5$ und $R^6$ die bei Formel (I) angegebene Bedeutung haben und n für 0 oder 1

steht, hydriert, in einer zweiten Stufe diese aminogruppenhaltigen N,N-Diarylcarbamoylfluoride mit Phosgen zu den entsprechenden Isocyanaten der Formel

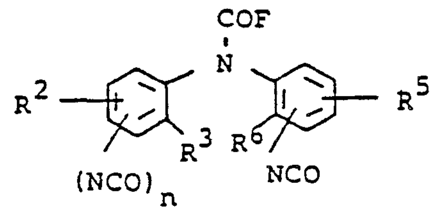
(XI)

worin die Reste $R^2$, $R^3$, $R^5$ und $R^6$ und n die vorstehend angegebene Bedeutung haben umsetzt, und diese in einer dritten Stufe mit Aminen der Formel

$$HNR^{13}R^{14} \quad (XII)$$

in der die Reste $R^{13}$ und $R^{14}$ die vorstehend angegebene Bedeutung haben, gegebenenfalls in einem inerten organischen Lösungsmittel, bei einer Temperatur von 0 bis 180 °C zu den Harnstoffen der Formel (IX) umsetzt.

Als Lösungsmittel seien beispielsweise für die Hydrierung der ersten Stufe Alkohole wie Methanol, Propanol, n-Butanol und tert.-Butanol und Ether wie Tetrahydrofuran und Dioxan genannt.

Bevorzugt werden Methanol und/oder Ethanol eingesetzt.

Als Hydrierungskatalysatoren können beispielsweise Metall- oder Edelmetallkatalysatoren wie Platin, Platinoxid, Palladium, Palladiumoxid, Raney-Nickel, Nickel, Raney-Kobalt, Palladium-Bariumcarbonat und Platin-Barium-sulfat verwendet werden, bevorzugt werden Platin, Palladium und Raney-Nickel eingesetzt.

Die Hydrierung kann beispielsweise bei einem Druck von 1 bis 50 bar, bevorzgut von 1 bis 40, besonders bevorzugt von 3 bis 30 bar ausgeführt werden.

Die Temperatur bei der Hydrierung kann im allgemeinen 20 bis 100 °C, bevorzugt 20 bis 60 °C, besonders bevorzugt 20 bis 40 °C betragen.

Die Hydrierung kann beispielsweise so durchgeführt werden, daß ein nitrogruppenhaltiges N,N-Diarylcarbamoylfluorid in einem organischen Lösungsmittel gelöst wird und unter Zusatz eines Hydrierungskatalysators hydriert wird. Das erhaltene aminogruppenhaltige N,N-Diarylcarbamoylfluorid kann nach einem der üblichen Verfahren, beispielsweise Kristallisation, Destillation und/oder Chromatographie isoliert und/oder gereinigt werden.

Die Phosgenierung der aminogruppenhaltigen N,N-Diarylcarbamoylfluoride der zweiten Stufe kann beispielsweise in Analogie zu Houben-Weyl 4. Auflage 1952, Band VIII, Seite 120 erfolgen, indem das Amin in einem inerten organischen Lösungsmittel, beispielsweise Toluol, Xylol oder Chlorkohlenwasserstoffen wie Chlorbenzol und Dichlorbenzole gelöst wird und mit einem Überschuß an Phosgen in einem Temperaturbereich von 0 °C bis zur Siedetemperatur des eingesetzten Lösungsmittels umgesetzt wird.

Die Umsetzung der dritten Stufe, der Isocyanate mit Aminen, kann beispielsweise nach Houben-Weyl, 4. Auflage 1952, Band VIII, Seite 157 durchgeführt werden, indem man das Isocyanat in einem inerten organischen Lösungsmittel löst und in einem Temperaturbereich von 0 bis 180 °C mit Aminen der Formel (XII) umsetzt.

Es können beispielsweise 1 bis 1,5, bevorzugt 1,2, besonders bevorzugt 1 Mol Amin pro Mol Isocyanat eingesetzt werden.

Die Umsetzung kann bevorzugt bei einer Temperatur von 10 bis 150 °C, besonders bevorzugt 20 bis 100 °C durchgeführt werden.

Als inerte organische Lösungsmittel können beispielsweise aliphatische Kohlenwasserstoffe wie Pentan, Hexan, Heptan und Cyclohexan, halogenierte aliphatische Kohlenwasserstoffe wie Dichlormethan, Trichlormethan und Tetrachlormethan, aromatische und substituierte aromatische Kohlenwasserstoffe wie Benzol, Toluol, Xylol, Chlorbenzol und Dichlorbenzole eingesetzt werden. Bevorzugt werden aromatische und substituierte aromatische Kohlenwasserstoffe wie Benzol, Toluol, Xylol, Chlorbenzol und Dichlorbenzole, besonders bevorzugt Toluol, Xylol und Chlorbenzol eingesetzt.

Bei der Umsetzung kann das Amin in Substanz zu der vorgelegten Lösung des isocyanatgruppenhaltigen Carbamoylfluorids zudosiert werden. Vorteilhafterweise kann das Amin im gleichen Lösungsmittel wie das isocyanatgruppenhaltige Carbamoylfluorid gelöst und im angegebenen Temperaturbereich zudosiert werden. Es kann aber auch zweckmäßig, sein, in umgekehrter Reihenfolge zu verfahren. Die Isolierung und Reinigung der Harnstoffe kann nach den allgemein üblichen Methoden, beispielsweise Kristallisation, Destillation und/oder Chromatographie erfolgen.

Die durch die erfindungsgemäße Verwendung der N,N-Diarylcarbamoyl-fluoride herstellbaren Harnstoffe der Formel (IX) sowie die dabei auftretenden und isolierbaren aminogruppenhaltigen Verbindungen der Formel (X) und die Isocyanate der Formel (XI) sind neu. Die Erfindung umfaßt auch die neuen Harnstoffe der Formel (IX).

Die erfindungsgemäßen Verfahren zur Herstellung von N,N-Diarylcarbamoyl-fluoriden haben gegenüber den bekannten Verfahren mehrere Vorteile. So kann das bekannte N,N-Diphenylcarbamoyl-fluorid nach den erfindungsgemäßen Verfahren in höheren Ausbeuten und in einer einfacheren Reaktionsfolge hergestellt werden. Darüberhinaus können N,N-Diarylcarbamoyl-fluoride hergestellt werden, deren Arylreste Substituenten tragen. Außerdem kann das Verfahren auf heterocyclische Systeme ausgedehnt werden.

Überraschend war die Beständigkeit der N,N-Diarylcarbamoyl-fluoride unter den Umsetzungsbedingungen der aromatischen Substitutionsreaktionen, wie den Nitrierungsreaktionen, sowie unter den Hydrierungsbedingungen.

Carbamidsäurehalogenide wirken als Chymotrypsin-Inaktivatoren, indem sie nucleophil spezifisch mit dem Chymotrypsin reagieren. Mit ihnen wird der Chymotrypsin-Anteil, der zur Verdauung von Eiweiß benötigt wird, im Darm beeinflußt (JACS *85*, 348 (1963), Biochemistry 3, 926 (1964), Analytical Biochemistry *33*, 318 (1970)).

Darüber hinaus zeigen die beschriebenen neuen Harnstoffe insektizide und herbizide Eigenschaften.

## Beispiele

1) Diphenylcarbamoylchlorid

34 g (0,2 Mol) Diphenylamin werden in 150 ml Toluol, dem 25 g Pyridin zugesetzt sind, gelöst. Bei einer Innentemperatur von 100 °C werden in ca. 60 Min. 30 g Phosgen eingeleitet. Mit Stickstoff wird in der Hitze ausgeblasen, nach Abkühlen vom ausgefallenen Pyridinhydrochlorid abgesaugt, das Filtrat eingeengt und mit Methylenchlorid über Kieselgel filtriert. Nach Einengen und Kristallisation aus Essigester erhält man 42,5 g (92 %) vom Fp. : 79-80 °C.

Analog werden die Ansätze 1a, 1b und 1c ausgeführt.

1a) p-Nitrodiphenylcarbamoylchlorid

| | | |
|---|---|---|
| Einsatzmengen : | 43 g | p-Nitrodiphenylamin |
| | 250 ml | Toluol |
| | 25 g | Pyridin |
| | 30 g | Phosgen |

Ausbeute : 48 g (86 %), Fp. : 113-114 °C (Essigester).

1b) Carbazolcarbamoylchlorid

| | | |
|---|---|---|
| Einsatzmengen : | 34 g | Carbazol |
| | 200 ml | Toluol |
| | 25 g | Pyridin |
| | 30 g | Phosgen |

Ausbeute : 37 g (70 %), Fp. : 90-91 °C (Toluol).

1c) Phenothiazincarbamoylchlorid

| | | |
|---|---|---|
| Einsatzmengen : | 40 g | Phenothiazin |
| | 250 ml | Toluol |
| | 25 g | Pyridin |
| | 30 g | Phosgen |

Ausbeute : 32 g (62 %), Fp. : 156-158°C (Ether).

2) Diphenylcarbamoylfluorid

Zu 300 ml wasserfreiem Fluorwasserstoff in einem $V_4$A-Rührautoklaven tropft man bei − 5 °C bis 0 °C eine Lösung von 173 g (0,75 Mol) Diphenylcarbamoylchlorid in 300 ml Methylenchlorid. Anschließend wird 60 Min. auf 50 °C bei einem Druck von 3 bar erhitzt. Dann werden nach Abkühlen und Entspannen die flüchtigen Bestandteile im Wasserstrahlvakuum bei einer Temperatur bis 50 °C entfernt und der Rückstand aus Ethanol umkristallisiert.

Ausbeute : 131 g (81 %), Fp. : 80-82 °C Analog werden die Ansätze 2a, 2b und 2c ausgeführt.

2a) p-Nitrodiphenylcarbamoylfluorid

| | | |
|---|---|---|
| Einsatzmengen : | 300 ml | Fluorwasserstoff |
| | 207 g | p-Nitrodiphenylcarbamoylchlorid |
| | 300 ml | $CH_2Cl_2$ |

Ausbeute : 156 g (80 %), Fp. : 93-94 °C (Ethanol).

2b) Carbazolcarbamoylfluorid

| | | |
|---|---|---|
| Einsatzmengen : | 300 ml | Fluorwasserstoff |
| | 172 g | Carbazolcarbamoylchlorid |
| | 300 ml | $CH_2Cl_2$ (Dichlormethan) |

Ausbeute : 123 g (77 %), Fp. : 95-97 °C.

2c) Phenothiazincarbamoylfluorid

| | | |
|---|---|---|
| Einsatzmengen : | 300 ml | Fluorwasserstoff |
| | 195 g | Phenothiazincarbamoylchlorid |
| | 300 ml | $CH_2Cl_2$ (Dichlormethan) |

Ausbeute : 133 g (72 %), Fp. : 98-100 °C.

3a) N-(4-Nitrophenyl)-N-phenylformamid

214 g (1 Mol) 4-Nitrodiphenylamin werden mit 170 g Zinkchlorid in 230 g Ameisensäure zwei Stunden am Rückfluß erhitzt. Nach Erkalten wird in 2 l Wasser gegossen, mit Chloroform extrahiert, über Magnesiumsulfat getrocknet, das Lösungsmittel im Vakuum abdestilliert und der ölige Rückstand mit 350 ml Ethanol verrührt. Das auskristallisierte Produkt wird abgesaugt und getrocknet.

Ausbeute : 210 g (87 %), Fp. : 78-79 °C.

3b) N-(4-Nitrophenyl)-N-phenylthioformamid

242 g (1 Mol) N-(4-Nitrophenyl)-N-phenylformamid werden mit 90 g Phosphorpentasulfid in 700 ml Pyridin zwei Stunden am Rückfluß erhitzt. Nach Erkalten wird in 2 l Wasser gegossen, das ausgefallene Produkt abgesaugt und aus Toluol umkristallisiert.

Ausbeute : 183 g (71 %), Fp. : 115-116 °C.

3c) Synthese von p-Nitrophenyl-N-phenylcarbamidsäurefluorid über die Fluorierung von N-Trichlormethyl-N-(4-Nitrophenyl)-N-phenylamin zu N-(Dichlorfluormethyl)-N-(4-Nitrophenyl)-N-phenylamin und dessen Verseifung mit Wasser

In eine Lösung von 258 g (1 Mol) N-(4-Nitrophenyl)-N-phenylthioformamid in 500 ml Tetrachlormethan leitet man in der Siedehitze während etwa 12 Stunden 210 g (3 Mol) Chlor ein. Anschließend werden die flüchtigen Bestandteile im Wasserstrahlvakuum abdestilliert, das verbleibende N-(4-Nitrophenyl)-N-phenyl-N-trichloramin in 350 ml abs. Chlorbenzol aufgenommen, mit 60 g wasserfreiem Kaliumfluorid versetzt und 3 Stunden lang zum Rückfluß erhitzt. Nach dem Abkühlen wird von den Salzen abfiltriert und das Filtrat unter Eiskühlung bei 0 °C bis + 5 °C tropfenweise mit 20 ml Wasser versetzt. Nach dem Abdestillieren der flüchtigen Bestandteile wird der verbleibende Rückstand aus Ethanol umkristallisiert.

Ausbeute : 166 g (64 %), Fp. : 93-94 °C.

4) Synthese des N-(4-Nitrophenyl)-N-phenylcarbamidsäurechlorids durch Verseifung von N-(4-Nitrophenyl)-N-phenyl-N-trichloramin

In eine Lösung von 258 g (1 · Mol) N-(4-Nitrophenyl)-N-phenylthioformamid in 500 ml Tetrachlormethan leitet man in der Siederhitze 210 g (3 Mol) Chlor in etwa 12 Stunden ein. Anschließend werden die flüchtigen Bestandteile im Wasserstrahlvakuum abdestilliert und das als Rückstand verbleibende N-(4-Nitrophenyl)-N-phenyl-N-trichloramin in 500 ml abs. Dichlormethan aufgenommen. Dazu tropft man unter Eiskühlung bei 0 °C bis + 10 °C 20 ml Wasser, destilliert das Lösungsmittel ab und kristallisiert den Rückstand aus Essigester um. 168 g (61 %, bezogen auf eingesetztes Thioformamid) vom Fp. : 113 °C.

5) Diphenylcarbamidsäurefluorid aus Diphenylamin und Monofluor-chlorphosgen

16,9 g (0,1 Mol) Diphenylamin werden in 80 ml Toluol, dem 10 g Pyridin zugesetzt sind, gelöst. Bei einer Innentemperatur von 80 bis 85 °C werden in etwa 45 Min. 10 g Monofluor-chlorphosgen eingeleitet. Nach dem Erkalten wird das ausgefallene Pyridinhydrochlorid abfiltriert, die flüchtigen Bestandteile des Filtrats im Wasserstrahlvakuum abdestilliert und der Rückstand mit Dichlormethan über Kieselgel chromatographisch filtriert. Nach dem Einengen wird der feste Rückstand aus Ethanol umkristallisiert. 17,0 g (79 %), Fp. : 80-82 °C.

6) N,N-Bis(4-nitrophenyl)-carbamoylfluorid

Zu 10 g p-Nitrodiphenylcarbamoylfluorid in 25 ml Essigsäureanhydrid tropft man bei 0° bis 5 °C ein Gemisch aus 5 g conz. Schwefelsäure und 5 g conz. Salpetersäure. Es wird 2 h bei dieser Temperatur, anschließend 1 h bei Raumtemperatur nachgerührt. Das Reaktionsgemisch wird auf 60 ml Methylenchlorid gegossen, anschließend mit 100 ml Wasser versetzt. Es wird mit Methylenchlorid extrahiert, mit Wasser gewaschen, mit Magnesiumsulfat getrocknet und eingeengt. Der braune Rückstand wird aus Toluol umkristallisiert.

Ausbeute : 8 g (67 %), Fp. : 170-172 °C.

7) N-(4-Aminophenyl)-N-phenylcarbamoylfluorid

130 g (0,5 Mol) p-Nitrodiphenylcarbamoylfluorid werden in 800 ml abs. Ethanol gelöst und an 20 g Raney-Nickel bei 40 °C und einem Wasserstoffdruck von 30 bar 4 h hydriert. Nach Entspannen und Abfiltrieren des Katalysators wird eingeengt und über Kieselgel mit Cyclohexan : Methylenchlorid 3 : 1 filtriert. Nach Einengen und Umkristallisieren aus Isopropanol erhält man 93 g (81 %), Fp. : 154 °C.

8) N-(4-Isocyanatophenyl)-N-phenylcarbamoylfluorid

In 400 ml Dichlorbenzol werden bei − 10 °C 80 g Phosgen einkondensiert. Bei 0 °C tropft man anschließend in etwa 40 Min. eine heiße Lösung von 23 g (0,1 Mol) N-(4-Aminophenyl)-N-phenylcarbamoylfluorid in 100 ml Dichlorbenzol zu. Bei einer Innentemperatur von 130 °C werden weitere 70 g Phosgen bis zum Entstehen einer klaren Lösung eingeleitet. Nach Abdestillieren des Lösungsmittels im Wasserstrahlvakuum wird der Rückstand im Ölpumpenvakuum destilliert.

Ausbeute : 24 g (94 %), Kp : 166-170 °C/0,8 Torr, Fp. : 67 °C.

9) Umsetzung von N-(4-Isocyanatophenyl)-N-phenylcarbamoylfluorid mit Anilin zum Harnstoff

25,6 g (0,1 Mol) N-(4-Isocyanatophenyl)-N-phenylcarbamoylfluorid werden in 150 ml Dichlormethan gelöst. Dazu tropft man 9,3 g (0,1 Mol) Anilin in 50 ml Dichlormethan. Es werden 60 Minuten bei 30 °C nachgerührt, die flüchtigen Bestandteile im Wasserstrahlvakuum entfernt und der Rückstand aus Essigester umkristallisiert. Man erhält 27,4 g (78 %) vom Fp. : 85 °C.

10) Diphenylcarbamidsäurefluorid aus Diphenylamin und Difluorphosgen

16,9 g (0,1 Mol) Diphenylamin werden in 80 ml Toluol, dem 10 g Pyridin zugesetzt sind, gelöst. Bei einer Innentemperatur von 80 bis 85 °C werden in etwa 45 Minuten 10 g Difluorphosgen eingeleitet. Nach dem Erkalten wird das ausgefallene Pyridinhydrochlorid abfiltriert, die flüchtigen Bestandteile des Filtrats im Wasserstrahlvakuum abdestilliert und der Rückstand mit Dichlormethan über Kieselgel chromatografisch filtriert. Nach dem Einengen wird der feste Rückstand aus Ethanol umkristallisiert.

Ausbeute : 14,8 g (69 %), Fp. : 80 bis 82 °C.

**Patentansprüche**

1. N,N-Diarylcarbamoylfluoride der Formel

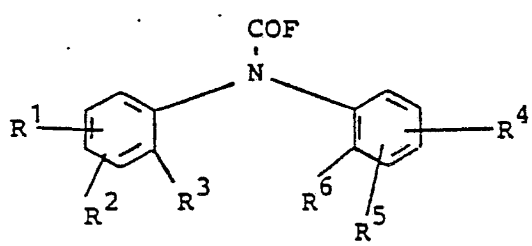

(I)

in der

$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,

$R^2$, $R^3$, $R^5$ und $R^6$ unabhängig voneinander Wasserstoff, Halogen, Alkyl oder CN bedeuten und weiterhin

$R^3$ und $R^6$ gemeinsam auch eine einfache Bindung, Sauerstoff, Schwefel, $CH_2$, CO oder Alkyliminoalkyliden bedeuten können,
wobei mindestens einer der Reste $R^1$ bis $R^6$ verschieden von Wasserstoff ist.

2. N,N-Diarylcarbamoylfluoride gemäß Anspruch 1, dadurch gekennzeichnet, daß sie der Formel

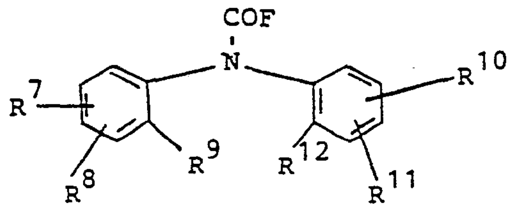

(II)

entsprechen, in der
$R^7$ und $R^{10}$ unabhängig voneinander Wasserstoff, $NO_2$, Halogen, Alkyl, Halogenalkyl, Aryl, mit Halogen und/oder $NO_2$ substituiertes Aryl, oder CN bedeuten,
$R^8$, $R^9$, $R^{11}$ und $R^{12}$ unabhängig voneinander Wasserstoff, Halogen oder Alkyl bedeuten und weiterhin
$R^9$ und $R^{12}$ gemeinsam auch eine einfache Bindung, Sauerstoff, Schwefel, $CH_2$ oder CO bedeuten, wobei mindestens einer der Reste $R^7$ bis $R^{12}$ verschieden von Wasserstoff ist.

3. Verfahren zur Herstellung von N,N-Diaryl-carb-amoyl-fluoriden der Formel

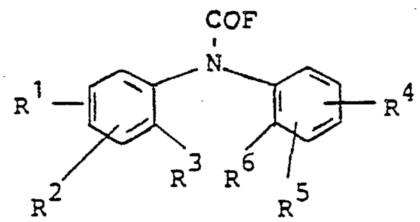

(Ia)

in der
$R^1$ und $R^4$ unabhängig voneinander Wasserstoff, $NO_2$, Halogen, Alkyl, Halogenalkyl, Aryl, mit Halogen und/oder $NO_2$ substituiertes Aryl, Dialkylamino oder CN bedeuten,
$R^2$, $R^3$, $R^5$ und $R^6$ unabhängig voneinander Wasserstoff, Halogen, Alkyl oder CN bedeuten und weiterhin
$R^3$ und $R^6$ gemeinsam auch eine einfache Bindung, Sauerstoff, Schwefel, $CH_2$, CO oder Alkyliminoalkyliden bedeuten, dadurch gekennzeichnet, daß man in einer ersten Stufe ein N,N-Diarylamin der Formel

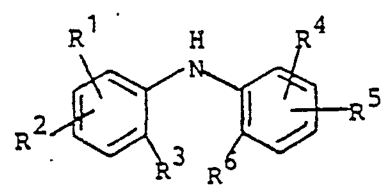

(III)

worin die Reste $R^1$ bis $R^6$ die bei Formel (Ia) angegebene Bedeutung haben, mit Phosgen, bei einer Temperatur von $-10$ bis 250 °C, gegebenenfalls in einem inerten organischen Lösungsmittel, gegebenenfalls in Gegenwart von mindestens molaren Mengen einer basisch reagierenden Substanz, gegebenenfalls unter erhöhtem Druck, zum N,N-Diarylcarbamoyl-chlorid der Formel

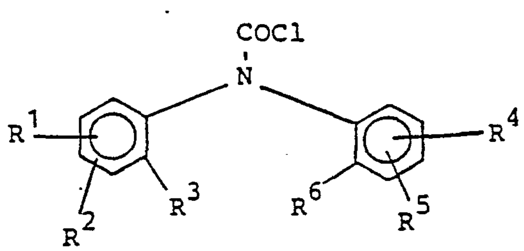

(IV)

worin die Reste $R^1$ bis $R^6$ die bei Formel (Ia) angegebene Bedeutung haben, umsetzt und das Umsetzungsprodukt in einem zweiten Schritt mit einem Fluorierungsmittel in einem Lösungsmittel bei einer Temperatur von $-20$ bis 300 °C, gegebenenfalls unter erhöhtem Druck, umsetzt,

wobei für den Fall, daß ein $NO_2$-haltiges N,N-Diarylcarbamoylfluorid aus einem $NO_2$-freien oder -ärmeren Ausgangsmaterial hergestellt werden soll, nach der Fluorierung mit den üblichen Nitrierungsmitteln nitriert wird und wobei weiter für den Fall, daß ein chlor- oder bromhaltiges N,N-Carbamoyl-fluorid aus einem chlor- oder brom- freien oder -ärmeren Ausgangsmaterial hergestellt werden soll, nach der Fluorierung in einem inerten Lösungsmittel, gegebenenfalls in Gegenwart von Halogenüberträgern, chloriert oder bromiert wird.

4. Verfahren zur Herstellung von N,N-Diaryl-carbamoyl-fluoriden der Formel (Ia) gemäß Anspruch 3, dadurch gekennzeichnet, daß man N,N-Diarylamine der Formel

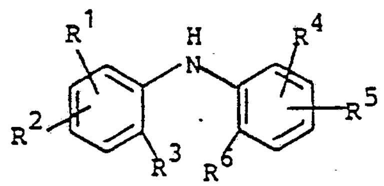

(III)

worin die Reste $R^1$ bis $R^6$ die in Anspruch 3 angegebene Bedeutung haben, mit Fluorphosgen bei einer Temperatur von − 10 bis 250 °C, gegebenenfalls in einem inerten organischen Lösungsmittel, gegebenenfalls in Gegenwart von mindestens molaren Mengen einer basisch reagierenden Substanz, gegebenenfalls unter erhöhtem Druck umsetzt,
wobei für den Fall, daß ein $NO_2$-haltiges N,N-Diarylcarbamoylfluorid aus einem $NO_2$-freien oder -ärmeren Ausgangsmaterial hergestellt werden soll, nach der Umsetzung mit den üblichen Nitrierungsmitteln nitriert wird und wobei weiter für den Fall, daß ein chlor- oder bromhaltiges N,N-Carbamoyl-fluorid aus einem chlor- oder brom- freien oder -ärmeren Ausgangsmaterial hergestellt werden soll, nach der Umsetzung in einem inerten Lösungsmittel, gegebenenfalls in Gegenwart von Halogenüberträgern, chloriert oder bromiert wird.

5. Verfahren zur Herstellung von N,N-Diarylcarbamoylfluoriden der Formel (Ia) gemäß Anspruch 3, dadurch gekennzeichnet, daß man in einem ersten Schritt N-Trichlormethyl-N,N-diarylamine der Formel

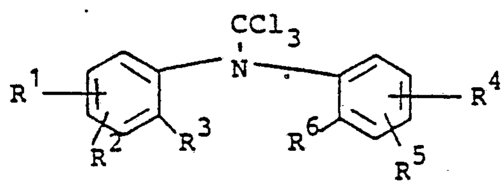

(V)

in der die Reste $R^1$ bis $R^6$ die in Anspruch 3 angegebene Bedeutung besitzen,
gegebenenfalls in Gegenwart eines inerten organischen Lösungsmittels, in einem Temperaturbereich von − 20 bis 100 °C mit Wasser umsetzt und in einer zweiten Stufe mit einem Fluorierungsmittel in einem Lösungsmittel bei einer Temperatur von − 20 bis 300 °C, gegebenenfalls unter erhöhtem Druck, umsetzt, wobei für den Fall, daß ein $NO_2$-haltiges N,N-Diarylcarbamoylfluorid aus einem $NO_2$-freien oder -ärmeren Ausgangsmaterial hergestellt werden soll, nach der Fluorierung mit den üblichen Nitrierungsmitteln nitriert wird und wobei weiter für den Fall, daß ein chlor- oder bromhaltiges N,N-Carbamoylfluorid aus einem chlor- oder bromfreien oder -ärmeren Ausgangsmaterial hergestellt werden soll, nach der Fluorierung in einem inerten Lösungsmittel, gegebenenfalls in Gegenwart von Halogenüberträgern, chloriert oder bromiert wird.

6. Verfahren zur Herstellung von N,N-Diarylcarbamoylfluoriden der Formel (Ia) gemäß Anspruch 3, dadurch gekennzeichnet, daß man N-Trichlormethyl-N,N-diarylformamide mit einem Fluorierungsmittel, gegebenenfalls in einem inerten organischen Lösungsmittel, gegebenenfalls bei erhöhter Temperatur, gegebenenfalls unter erhöhtem Druck, zu N-Dichlorfluormethyl-N,N-diarylaminen der Formel

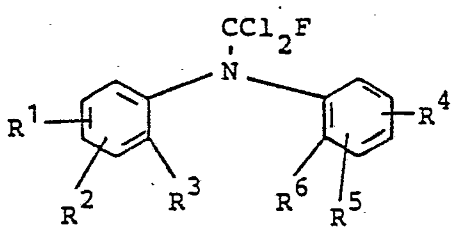

(VIII)

in der die Reste $R^1$ bis $R^6$ die bei Formel (Ia) in Anspruch 3 angegebene Bedeutung haben, fluoriert und in einem zweiten Reaktionsschritt gegebenenfalls in einem Lösungsmittel, gegebenenfalls bei einer Temperatur von − 20 bis 100 °C mit Wasser umsetzt, wobei für den Fall, daß ein $NO_2$-haltiges N,N-Diarylcarbamoyl-fluorid aus einem $NO_2$-freien oder -ärmeren Ausgangsmaterial hergestellt werden soll, nach

der Umsetzung mit einem üblichen Nitrierungsmittel nitriert wird und wobei für den Fall, daß ein chlor- oder bromhaltiges N,N-Carbamoyl-fluorid aus einem chlor- oder bromfreien oder -ärmeren Ausgangsmaterial hergestellt werden soll, nach der Umsetzung in einem inerten Lösungsmittel, gegebenenfalls in Gegenwart von Halogenüberträgern, chloriert oder bromiert wird.

7. Harnstoffe der Formel

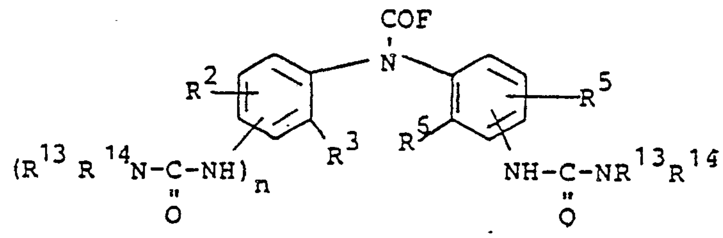

(IX)

in der die Reste $R^2$, $R^3$, $R^5$ und $R^6$ die bei Formel (I) in Anspruch 1 angegebene Bedeutung haben,

$R^{13}$ und $R^{14}$ unabhängig voneinander für Wasserstoff, Alkyl, Halogenalkyl, Aryl, mit Halogen und/oder $NO_2$ substituiertes Aryl stehen und für 0 oder 1 steht.

8. Verwendung der N,N-Diarylcarbamoylfluoride der Formel (I) gemäß Anspruch 1 zur Herstellung von Harnstoffen der Formel

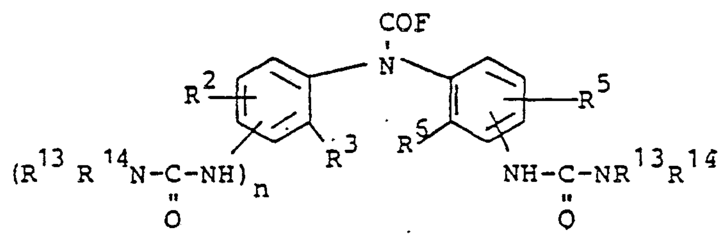

(IX)

in der die Reste $R^2$, $R^3$, $R^5$ und $R^6$ die bei Formel (I) in Anspruch 1 angegebene Bedeutung haben,

$R^{13}$ und $R^{14}$ unabhängig voneinander für Wasserstoff, Alkyl, Halogenalkyl, Aryl, mit Halogen und/oder $NO_2$ substituiertes Aryl stehen und

n für 0 oder 1 steht,

dadurch gekennzeichnet, daß man in einer ersten Stufe nitrogruppenhaltige N,N-Diarylcarbamoylfluoride der Formel (I) gemäß Anspruch 1 gegebenenfalls in Gegenwart eines organischen Lösungsmittels in Gegenwart eines geeigneten Hydrierungskatalysators mit Wasserstoff zu aminogruppenhaltigen N,N-Diarylcarbamoylfluoriden der Formel

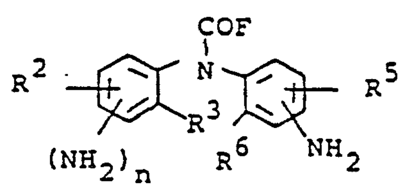

(X)

in der die Reste $R^2$, $R^3$, $R^5$ und $R^6$ die bei Formel (I) in Anspruch 1 angegebene Bedeutung haben und n für 0 oder 1 steht, hydriert, in einer zweiten Stufe diese aminogruppenhaltigen N,N-Diarylcarbamoylfluoride beispielsweise mit Phosgen zu den entsprechenden Isocyanaten der Formel

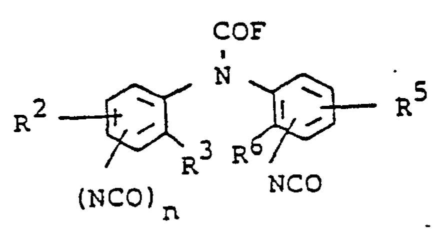

(XI)

worin die Reste $R^2$, $R^3$, $R^5$ und $R^6$ und n die vorstehend angegebene Bedeutung haben, und diese in einer dritten Stufe mit Aminen der Formel

$$HNR^{13}R^{14}$$

in der die Reste $R^{13}$ und $R^{14}$ die vorstehend angegebene Bedeutung haben, gegebenenfalls in einem inerten organischen Lösungsmittel bei einer Temperatur von 0 bis 180 °C zu den Harnstoffen der Formel

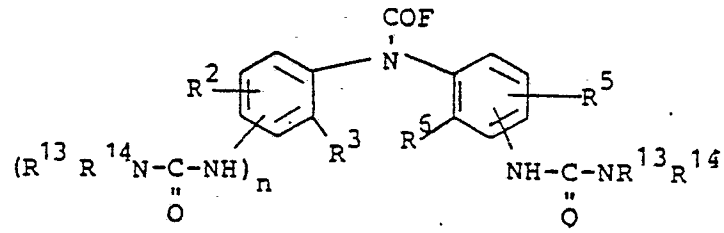

(IX)

worin die Reste $R^2$, $R^3$, $R^5$, $R^6$, $R^{13}$ und $R^{14}$ sowie n die vorstehend angegebene Bedeutung haben, umsetzt.

**Claims**

1. N,N-Diarylcarbamoyl fluorides of the formula

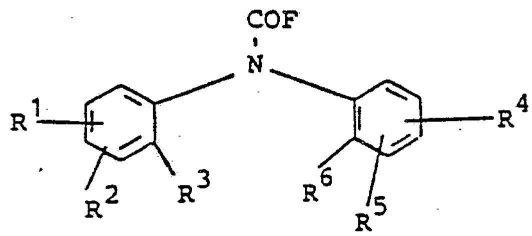

(I)

in which

$R^1$ and $R^4$ independently of one another denote hydrogen, $NO_2$, halogen, alkyl, halogenoalkyl, aryl, halogen- and/or $NO_2$-substituted aryl, dialkylamino or CN,

$R^2$, $R^3$, $R^5$ and $R^6$ independently of one another denote hydrogen, halogen, alkyl or CN and, furthermore,

$R^3$ and $R^6$ together can also denote a single bond, oxygen, sulphur, $CH_2$, CO or alkyliminoalkylidene, at least one of the radicals $R^1$ to $R^6$ being different from hydrogen.

2. N,N-Diarylcarbamoyl fluorides according to Claim 1, characterised in that they correspond to the formula

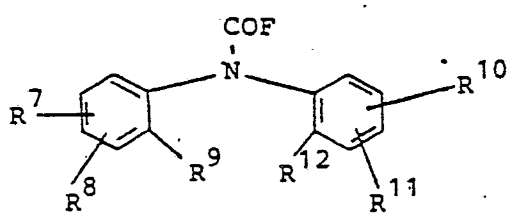

(II)

in which

$R^7$ and $R^{10}$ independently of one another denote hydrogen, $NO_2$, halogen, alkyl, halogenoalkyl, aryl, halogen- and/or $NO_2$-substituted aryl, or CN,

$R^8$, $R^9$, $R^{11}$ and $R^{12}$ independently of one another denote hydrogen, halogen or alkyl, and, furthermore,

$R^9$ and $R^{12}$ together also denote a single bond, oxygen, sulphur, $CH_2$ or CO, at least one of the radicals $R^7$ to $R^{12}$ being different from hydrogen.

3. Process for the preparation of N,N-diarylcarbamoyl fluorides of the formula

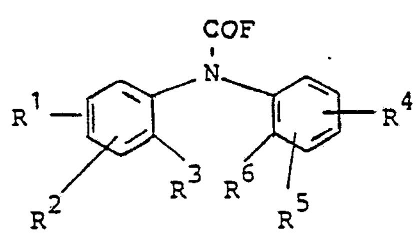

(Ia)

in which

$R^1$ and $R^4$ independently of one another denote hydrogen, $NO_2$, halogen, alkyl, halogenoalkyl, aryl, halogen- and/or $NO_2$-substituted aryl, dialkylamino or CN,

$R^2$, $R^3$, $R^5$ and $R^6$ independently of one another denote hydrogen, halogen, alkyl or CN and, furthermore,

$R^3$ and $R^6$ together also denote a single bond, oxygen, sulphur, $CH_2$, CO or alkyliminoalkylidene, characterised in that, in a first stage, an N,N-diarylamine of the formula

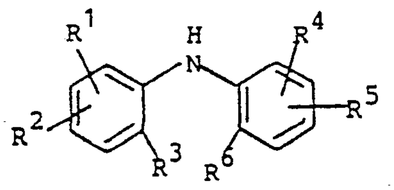

(III)

wherein the radicals $R^1$ to $R^6$ have the meaning indicated under formula (Ia), is reacted with phosgene at a temperature of – 10 to 250 °C, optionally in an inert organic solvent, optionally in the presence of at least molar quantities of a basic-reacting substance, and optionally under elevated pressure, to give N,N-diarylcarbamoyl chloride of the formula

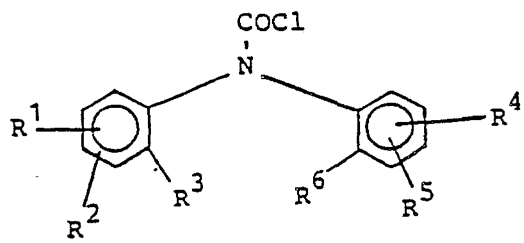

(IV)

wherein the radicals $R^1$ to $R^6$ have the meaning given under formula (Ia) and, in a second step, the reaction product is reacted with a fluorinating agent in a solvent at a temperature of – 20 to 300 °C, optionally under elevated pressure, wherein in the case where an $NO_2$-containing N,N-diarylcarbamoyl fluoride is to be prepared from an $NO_2$-free starting material or one having a lower content of $NO_2$, nitration is carried out with the customary nitrating agents after the fluorination, and wherein, furthermore, in the case where a chlorine- or bromine-containing N,N-carbamoyl fluoride is to be prepared from a chlorine- or bromine-free starting material or one having a lower content of chlorine or bromine, chlorination or bromination is carried out in an inert solvent, optionally in the presence of halogen carriers, after the fluorination.

4. Process for the preparation of N,N-diarylcarbamoyl fluorides of the formula (Ia) according to Claim 3, characterised in that N,N-diarylamines of the formula

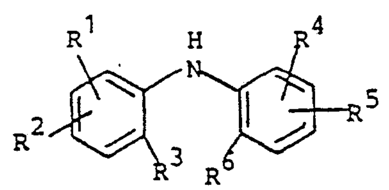

(III)

wherein the radicals $R^1$ to $R^6$ have the meaning given in Claim 3, are reacted with fluorophosgene at a temperature of – 10 to 250 °C, optionally in an inert organic solvent, optionally in the presence of at least molar quantities of a basic-reacting substance, and optionally under elevated pressure, wherein in the case where an NO-containing N,N-diarylcarbamoyl fluoride is to be prepared from an $NO_2$-free starting material or one having a lower content of $NO_2$, nitration is carried out with the customary nitrating agents, after the reaction, and wherein, furthermore, in the case where a chlorine- or bromine-containing N,N-carbamoyl fluoride is to be prepared from a chlorine- or bromine-free starting material or one having a low content of chlorine or bromine, chlorination or bromination is carried out in an inert solvent, optionally in the presence of halogen carriers, after the reaction.

5. Process for the preparation of N,N-diarylcarbamoyl fluorides of the formula (Ia) according to Claim 3, characterised in that, in a first step, N-trichloromethyl-N,N-diarylamines of the formula

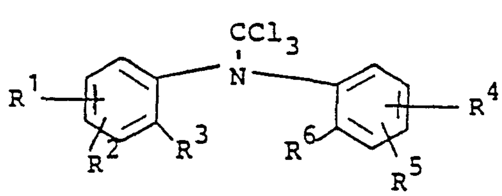

(V)

in which the radicals $R^1$ to $R^6$ have the meaning given in Claim 3, are reacted with water, optionally in the presence of an inert organic solvent, in a temperature range of − 20 to 100 °C, and, in a second stage, the product is reacted with a fluorinating agent in a solvent at a temperature of − 20 to 300 °C, optionally under elevated pressure, wherein, in the case where an $NO_2$-containing N,N-diarylcarbamoyl fluoride is to be prepared from an $NO_2$-free starting material or one having a lower content of $NO_2$, nitration is carried out with the customary nitrating agents after the fluorination and wherein, furthermore, in the case where a chlorine- or bromine-containing N,N-carbamoyl fluoride is to be prepared from a chlorine- or bromine-free starting material or one having a lower content of chlorine or bromine, chlorination or bromination is carried out in an inert solvent, optionally in the presence of halogen carriers, after the fluorination.

6. Process for the preparation of N,N-diarylcarbamoyl fluorides of the formula (Ia) according to Claim 3, characterised in that N-trichloromethyl-N,N-diarylformamides are fluorinated with a fluorinating agent, optionally in an inert organic solvent, optionally at an elevated temperature, and optionally under elevated pressure, to give N-dichlorofluoromethyl-N,N-diarylamines of the formula

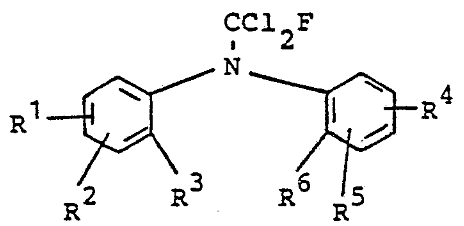

(VIII)

in which the radicals $R^1$ to $R^6$ have the meaning given under formula (Ia) in Claim 3, and, in a second reaction step, the reaction product is reacted with water, optionally in a solvent and optionally at a temperature of − 20 to 100 °C, wherein in the case where an $NO_2$-containing N,N-diarylcarbamoyl fluoride is to be prepared from an $NO_2$-free starting material or one having a lower content of $NO_2$, nitration is carried out with a customary nitrating agent after the reaction and wherein, in the case where a chlorine- or bromine-containing N,N-carbamoyl fluoride is to be prepared from a chlorine- or bromine-free starting material or one having a lower content of chlorine or bromine, chlorination or bromination is carried out in an inert solvent, optionally in the presence of halogen carriers, after the reaction.

7. Ureas of the formula

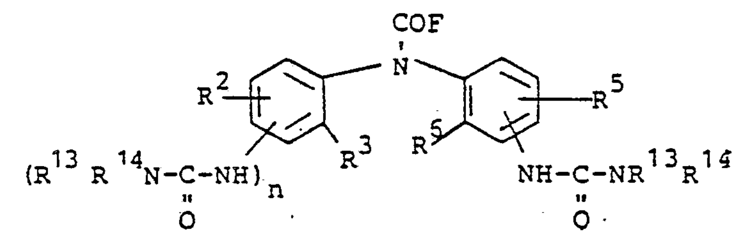

(IX)

in which

the radicals $R^2$, $R^3$, $R^5$ and $R^6$ have the meaning given under formula (I) in Claim 1,

$R^{13}$ and $R^{14}$ independently of one another represent hydrogen, alkyl, halogenoalkyl, aryl, or halogen, and/or $NO_2$-substituted aryl and

n represents 0 or 1.

8. Use of the N,N-diarylcarbamoyl fluorides of the formula (I) according to Claim 1 for the preparation of ureas of the formula

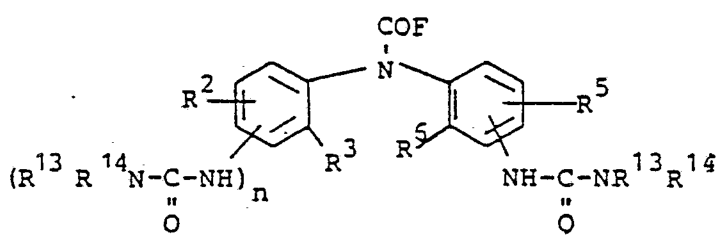

(IX)

in which

the radicals $R^2$, $R^3$, $R^5$ and $R^6$ have the meaning given under formula (I) in Claim 1,

$R^{13}$ and $R^{14}$ independently of one another represent hydrogen, alkyl, halogenoalkyl, aryl, or halogen- and/or $NO_2$-substituted aryl, and

n represents 0 or 1,

characterised in that, in a first stage, nitro-group-containing N,N-diarylcarbamoyl fluorides of the formula

(I) according to Claim 1 are hydrogenated with hydrogen, optionally in the presence of an organic solvent and in the presence of a suitable hydrogenation catalyst, to give amino-group-containing N,N-diarylcarbamoyl fluorides of the formula

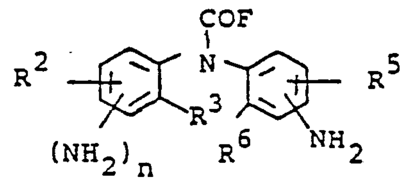

(X)

in which

the radicals $R^2$, $R^3$, $R^5$ and $R^6$ have the meaning given under formula (I) in Claim 1 and n represents 0 or 1,

in a second stage, these amino-group-containing N,N-diarylcarbamoyl fluorides are reacted, for example with phosgene, to give the corresponding isocyanates of the formula

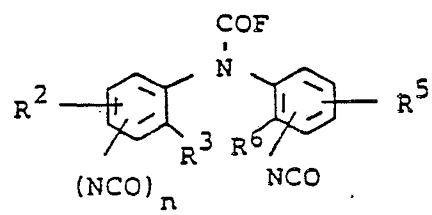

(XI)

wherein the radicals $R^2$, $R^3$, $R^5$ and $R^6$ and n have the aforementioned meaning, and these, in a third stage, are reacted with amines of the formula

$$HNR^{13}R^{14}$$

in which the radicals $R^{13}$ and $R^{14}$ have the aforementioned meaning, optionally in an inert organic solvent at a temperature of 0 to 180 °C to give the ureas of the formula

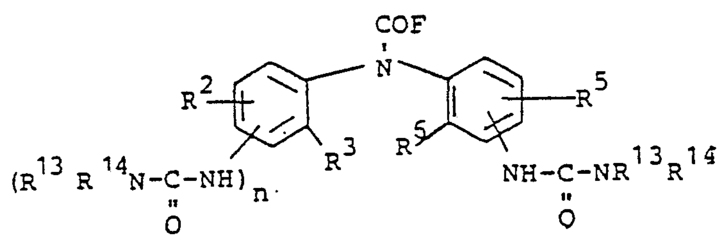

(IX)

wherein the radicals $R^2$, $R^3$, $R^5$, $R^6$, $R^{13}$ and $R^{14}$ and n have the aforementioned meaning.

**Revendications**

1. Fluorures de N,N-diarylcarbamoyle de formule

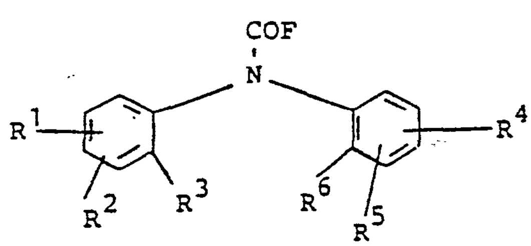

(I)

dans laquelle

$R^1$ et $R^4$ désignent, indépendamment l'un de l'autre, l'hydrogène, un groupe $NO_2$, un halogène, un groupe alkyle, halogénalkyle, aryle, aryle substitué avec un halogène et/ou un radical $NO_2$, dialkylamino ou CN,

$R^2$, $R^3$, $R^5$ et $R^6$ représentent, indépendamment l'un de l'autre, l'hydrogène, un halogène, un groupe alkyle ou un groupe CN et en outre

$R^3$ et $R^6$ peuvent aussi former ensemble une liaison simple, un atome d'oxygène, de soufre, un groupe $CH_2$, CO ou alkylimino-alkylidène, au moins l'un des restes $R^1$ à $R^6$ représentant autre chose que l'hydrogène.

2. Fluorures de N,N-diarylcarbamoyle suivant la revendication 1, caractérisés en ce qu'ils répondent à la formule

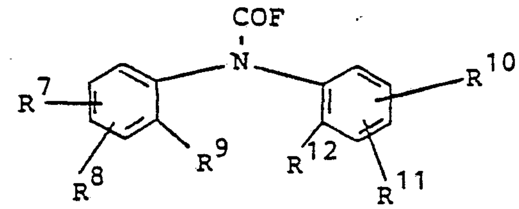

(II)

dans laquelle

$R^7$ et $R^{10}$ désignent, indépendamment l'un de l'autre, l'hydrogène, un groupe $NO_2$, un halogène, un groupe alkyle, halogénalkyle, aryle, aryle substitué avec un halogène et/ou un radical $NO_2$, ou CN,

$R^8$, $R^9$, $R^{11}$ et $R^{12}$ représentent, indépendamment l'un de l'autre, l'hydrogène, un halogène ou un groupe alkyle et en outre

$R^9$ et $R^{12}$ désignent ensemble également une liaison simple, un atome d'oxygène, un atome de soufre, un groupe $CH_2$ ou CO, au moins l'un des restes $R^7$ à $R^{12}$ représentant autre chose que de l'hydrogène.

3. Procédé de production de fluorures de N,N-diarylcarbamoyle de formule

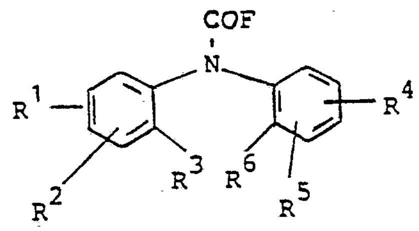

(Ia)

dans laquelle

$R^1$ et $R^4$ désignent, indépendamment l'un de l'autre, l'hydrogène, un groupe $NO_2$, un halogène, un groupe alkyle, halogénalkyle, aryle, aryle substitué avec un halogène et/ou un radical $NO_2$, dialkylamino ou CN,

$R^2$, $R^3$, $R^5$ et $R^6$ représentent, indépendamment l'un de l'autre, l'hydrogène, un halogène, un groupe alkyle ou un groupe CN et en outre

$R^3$ et $R^6$ peuvent aussi former ensemble une liaison simple, un atome d'oxygène, de soufre, un groupe $CH_2$, CO ou alkylimino-alkylidène,

caractérisé en ce qu'on fait réagir dans une première étape une N,N-diarylamine de formule

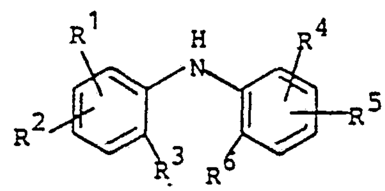

(III)

dans laquelle les restes $R^1$ à $R^6$ ont la définition indiquée à propos de la formule (Ia), avec du phosgène, à une température de − 10 à 250 °C, éventuellement dans un solvant organique inerte, le cas échéant en présence de quantités au moins molaires d'une substance à réaction basique, éventuellement sous pression élevée, pour former le chlorure de N,N-diarylcarbamoyle de formule

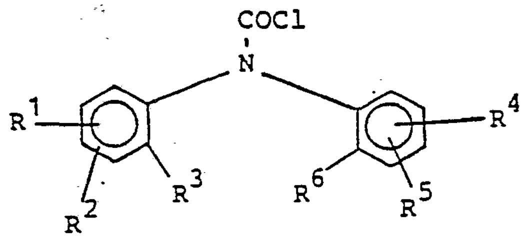

(IV)

dans laquelle les restes $R^1$ à $R^6$ ont la définition indiquée pour la formule (Ia) et on fait réagir le produit réactionnel dans une seconde étape avec un agent de fluoration dans un solvant à une température de

– 20 à 300 °C, éventuellement sous pression élevée, et au cas où l'on doit préparer un fluorure de N,N-diarylcarbamoyle porteur du groupe $NO_2$ à partir d'une matière de départ dépourvue de $NO_2$ ou assez pauvre en $NO_2$, on effectue la nitration avec les agents classiques de nitration après la fluoration et au cas où, en outre, on doit préparer un fluorure de N,N-carbamoyle contenant du chlore ou du brome, à partir d'une matière de départ dépourvue de chlore ou de brome ou assez pauvre en chlore ou en brome, on effectue la chloration ou la bromation après la fluoration dans un solvant inerte, éventuellement en présence de vecteurs d'halogènes.

4. Procédé de production de fluorures de N,N-diarylcarbamoyle de formule (Ia) suivant la revendication 3, caractérisé en ce qu'on fait réagir de N,N-diarylamines de formule

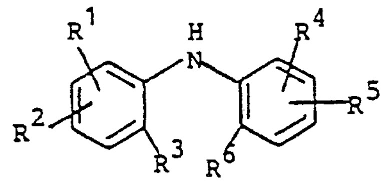

(III)

dans laquelle les restes $R^1$ à $R^6$ ont la définition indiquée dans la revendication 3, avec du fluorophosgène à une température de – 10 à 250 °C, éventuellement dans un solvant organique inerte, le cas échéant en présence de quantités au moins molaires d'une substance à réaction basique, éventuellement sous pression élevée, au cas où l'on doit préparer un fluorure de N,N-diarylcarbamoyle contenant $NO_2$ à partir d'une matière de départ dépourvue de $NO_2$ ou assez pauvre en $NO_2$, on effectue la nitration après la réaction avec les agents classiques de nitration et au cas où l'on doit en outre préparer un fluorure de N,N-carbamoyle contenant du chlore ou du brome à partir d'une matière de départ dépourvue de chlore ou en brome, on effectue la chloration ou la bromation après la réaction dans un solvant inerte, éventuellement en présence de vecteurs d'halogènes.

5. Procédé de production de fluorures de N,N-diarylcarbamoyle de formule (Ia) suivant la revendication 3, caractérisé en ce qu'on fait réagir dans une première étape des N-trichlorométhyl-N,N-diarylamines de formule

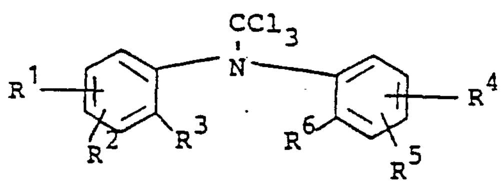

(V)

dans laquelle les restes $R^1$ à $R^6$ possèdent la définition indiquée dans la revendication 3, avec l'eau, éventuellement en présence d'un solvant organique inerte dans un intervalle de température de – 20 à 100 °C et dans une seconde étape, on les fait réagir avec un agent de fluoration dans un solvant à une température de – 20 à 300 °C, le cas échéant sous pression élevée, au cas où l'on doit préparer un fluorure de N,N-diarylcarbamoyle contenant $NO_2$ à partir d'une matière de départ dépourvue de $NO_2$ ou assez pauvre en $NO_2$, on effectue une nitration après la fluoration avec les agents classiques de nitration et au cas où l'on doit en outre obtenir un fluorure de N,N-carbamoyle contenant du chlore ou du brome à partir d'une matière de départ dépourvue de chlore ou de brome ou assez pauvre en chlore ou en brome, on effectue la chloration ou la bromation après la fluoration dans un solvant inerte, éventuellement en présence de vecteurs d'halogènes.

6. Procédé de production de fluorures de N,N-diarylcarbamoyle de formule (Ia) suivant la revendication 3, caractérisé en ce qu'on fluore des N-trichlorométhyl-N,N-diarylformamides avec un agent de fluoration, le cas échéant dans un solvant organique inerte, éventuellement à température élevée et, le cas échéant, sous pression élevée, pour former des N-dichlorofluorométhyl-N,N-diaryl-amines de formule

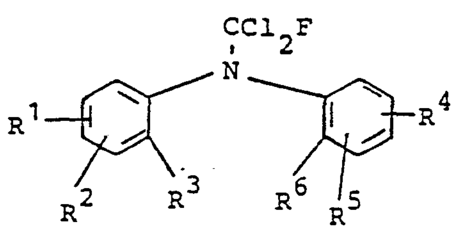

(VIII)

dans laquelle les restes $R^1$ à $R^6$ ont la définition indiquée dans la revendication 3 pour la formule (Ia) et

dans une seconde étape réactionnelle, on les fait réagir avec de l'eau, éventuellement dans un solvant, le cas échéant à une température de – 20 à 100 °C, au cas où l'on doit préparer un fluorure de N,N-diarylcarbamoyle contenant le groupe $NO_2$ à partir d'une matière de départ dépourvue de $NO_2$ ou assez pauvre en $NO_2$, on effectue la nitration après la réaction avec un agent classique de nitration et au cas où on doit préparer un fluorure de N,N-carbamoyle contenant du chlore ou du brome à partir d'une matière de départ dépourvue de chlore ou de brome ou assez pauvre en chlore ou en brome, on effectue la chloration ou la bromation après la réaction dans un solvant inerte, éventuellement en présence de vecteurs d'halogènes.

7. Urées de formule

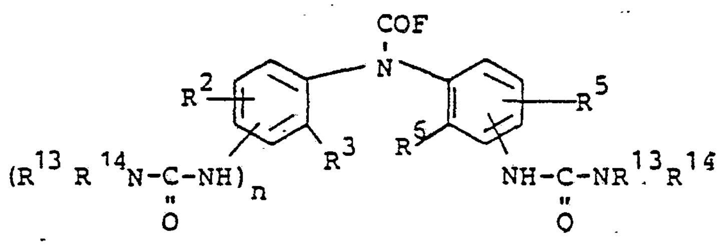

(IX)

dans laquelle les restes $R^2$, $R^3$, $R^5$ et $R^6$ ont la définition indiquée pour la formule (I) dans la revendication 1, $R^{13}$ et $R^{14}$ représentent, indépendamment l'un de l'autre, l'hydrogène, un groupe alkyle, halogénalkyle, aryle, aryle substitué avec un halogène et/ou un groupe $NO_2$ et n a la valeur 0 ou 1. .

8. Utilisation des fluorures de N,N-diarylcarbamoyle de formule (I) suivant la revendication 1 pour la production d'urées de formule

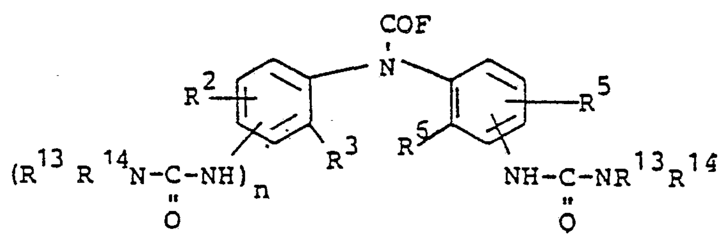

(IX)

dans laquelle les restes $R^2$, $R^3$, $R^5$ et $R^6$ ont la définition indiquée pour la formule (I) dans la revendication 1, $R^{13}$ et $R^{14}$ représentent, indépendamment l'un de l'autre, l'hydrogène, un groupe alkyle, halogénalkyle, aryle, aryle substitué avec un halogène et/ou un groupe $NO_2$ et n a la valeur 0 ou 1, caractérisée en ce qu'on hydrogène dans une première étape des fluorures de N,N-diarylcarbamoyle porteurs de groupes nitro de formule (I) suivant la revendication 1, éventuellement en présence d'un solvant organique, en présence d'un catalyseur d'hydrogénation approprié, avec de l'hydrogène pour former des fluorures de N,N-diarylcarbamoyle porteurs de groupes amino de formule

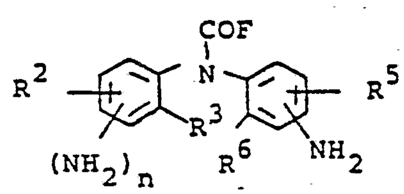

(X)

dans laquelle les restes $R^2$, $R^3$, $R^5$ et $R^6$ ont la définition indiquée pour la formule (I) dans la revendication 1 et n a la valeur 0 ou 1, on fait réagir dans une seconde étape ces fluorures de N,N-diarylcarbamoyle porteurs de groupes amino, par exemple avec le phosgène pour former les isocyanates correspondants de formule

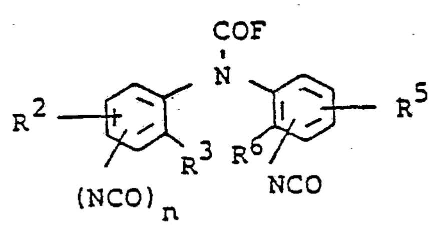

(XI)

dans laquelle les restes $R^2$, $R^3$, $R^5$ et $R^6$ et n ont la définition indiquée ci-dessus et on les fait réagir dans une troisième étape avec des amines de formule

$$HNR^{13}R^{14}$$

dans laquelle les restes $R^{13}$ et $R^{14}$ ont la définition indiquée ci-dessus, éventuellement dans un solvant organique inerte à une température de 0 à 180 °C pour former les urées de formule

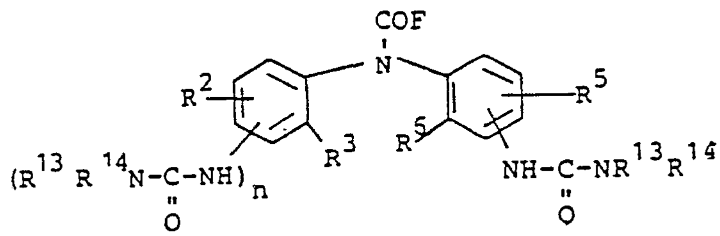

(IX)

dans laquelle les restes $R^2$, $R^3$, $R^5$, $R^6$, $R^{13}$ et $R^{14}$ ainsi que n ont la définition indiquée ci-dessus.